(19) Europäisches Patentamt
European Patent Office
Office européen des brevets

(11) **EP 4 129 354 A1**

(12) **EUROPEAN PATENT APPLICATION**
published in accordance with Art. 153(4) EPC

(43) Date of publication:
**08.02.2023 Bulletin 2023/06**

(21) Application number: **21780375.8**

(22) Date of filing: **31.03.2021**

(51) International Patent Classification (IPC):
**A61M 1/02** (1968.09)     **A61M 1/16** (1985.01)

(52) Cooperative Patent Classification (CPC):
**A61M 1/02; A61M 1/16**

(86) International application number:
**PCT/JP2021/014033**

(87) International publication number:
**WO 2021/201172 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2020  JP 2020064252**

(71) Applicant: **Asahi Kasei Medical Co., Ltd.
Tokyo 100-0006 (JP)**

(72) Inventor: **OISHI, Teruhiko
Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **BLOOD PURIFICATION SYSTEM, CONTROLLING METHOD, CONTROLLING PROGRAM, LEARNING DEVICE, AND LEARNING METHOD**

(57)     Provided are a blood purification system, etc., to enable more achieve more efficient purification of blood. A blood purification system includes a line through which a liquid containing blood or filtrate flows, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a parameter acquisition module to input the detected blood information into a learning model trained to output predetermined control parameters when predetermined blood information is input, and acquires control parameters outputted from the learning model, and a control module to control the liquid control mechanism based on the acquired control parameters.

FIG. 3

**Description**

FIELD

The present disclosure relates to a blood purification system, a control method, a control program, a learning apparatus and a learning method.

BACKGROUND

Blood purification systems used for dialysis are constructed with multiple systems in a dialysis room provided in a medical facility such as a hospital, with blood purification being carried out for many patients in the dialysis room. In such a dialysis room, a server (central control means) is installed, which stores management data for patients undergoing blood purification (such as patient body weight and blood pressure), with the management data being sent to individual blood purification systems for display.

When a patient is to undergo dialysis, first the pre-dialysis body weight and blood pressure are measured using a weight scale or sphygmomanometer, and are sent to the central control means and stored as unique information for the patient. Based on the patient's unique information, the central control means determines the condition of the patient by computation with a predetermined formula, and the determined condition is transmitted to a blood purification system to allow optimal blood purification for each patient.

PTL 1, for example, discloses a blood purification system having multiple monitoring devices installed in the dialysis room of a medical facility such as a hospital.

CITATION LIST

PATENT LITERATURE

Patent Literature 1: Japanese Unexamined Patent Publication No. 2012-249748

SUMMARY

Achieving more efficient purification of blood is a desired goal for blood purification systems.

An object of a blood purification system, a control method, a control program, a learning apparatus or a learning method is to enable more achieve more efficient purification of blood.

According to some embodiments, a blood purification system includes a line through which a liquid containing blood or filtrate flows, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a parameter acquisition module to input the blood information detected by the detector into a learning model trained to output predetermined control parameters when predetermined blood information is input, and acquires control parameters outputted from the learning model, and a control module to control the liquid control mechanism based on the control parameters acquired by the parameter acquisition module. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

Preferably, the learning model has an action value function using the blood information as a state, and control based on the control parameter as an action, and the action value function is updated based on a reward set so as to be greater as change in the blood information is smaller.

Preferably, the liquid control mechanism includes a magnetic regulator to apply a magnetic field to blood in a predetermined direction, a pump to control the flow of liquid in the line, or a resistance member to apply resistance to the line, and the control parameters include at least one from among magnetic field strength applied by the magnetic regulator, drive amount of the pump and a value of resistance applied by the resistance member.

Preferably, the blood purification system includes a storage to store the learning model in association with product data for the blood purification device, data relating to blood flowing through the line, clearance data by the blood purification system, causative substance removal, or data related to antithrombogenicity.

Preferably, the blood purification system includes a generation module to control the liquid control mechanism based on a specific control parameter, and generate the learning model based on the blood information detected by the detector before and after controlling the liquid control mechanism, and the specific control parameter.

Preferably, the blood purification system is an extracorporeal circulation blood purification system.

Preferably, the blood purification system carries out continuous hemodialysis filtration, continuous hemofiltration, continuous hemodialysis or apheresis.

According to some embodiments, a control method of a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, includes inputting the blood information detected by the detector into a learning model trained to output predetermined control parameters when predetermined blood information is input, and acquiring control parameters outputted from the learning model, and controlling the liquid control mechanism based on the acquired control parameters. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

According to some embodiments, a control program of a computer included in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, causes the computer to execute a process. The process includes inputting the blood information detected by the detector into a learning model trained to output predetermined control parameters when predetermined blood information is input, and acquiring control parameters outputted from the learning model, and controlling the liquid control mechanism based on the acquired control parameters. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

According to some embodiments, a learning apparatus includes a data acquisition module to acquire, in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters, a generation module to generate a learning model trained to output predetermined control parameters when predetermined blood information is input, using the sets acquired by the a data acquisition module, and an output control module to output information relating to the learning model. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

Preferably, the learning apparatus further includes a communication device to communicate with multiple blood purification systems, and the data acquisition module acquires the sets by receiving it from the multiple blood purification systems via the communication device.

Preferably, the data acquisition module controls the liquid control mechanism based on specific control parameters, and acquires blood information detected by the detector before and after control of the liquid control mechanism, based on the specific control parameter, to acquire the sets.

According to some embodiments, a learning method includes acquiring, by a computer, in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters, generating, by the computer, a learning model trained to output predetermined control parameters when predetermined blood information is input, using the acquired sets, and outputting, by the computer, information relating to the learning model. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

According to some embodiments, a control program of a computer, causes the computer to execute a process. The process includes acquiring in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters, generating a learning model trained to output predetermined control parameters when predetermined blood information is input, using the acquired sets, and outputting information relating to the learning model. The blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

The blood purification system, the control method, the control program, the learning apparatus and the learning method can achieve more efficient purification of blood.

The object and advantages of the invention will be realized and attained by means of the elements and combinations, in particular, described in the claims. It is to be understood that both the foregoing general description and the following detailed description are exemplary and explanatory, and are not restrictive of the invention, as claimed.

BRIEF DESCRIPTION OF DRAWINGS

Fig. 1 is a diagram showing the general configuration of a management system 100 according to an embodiment.
Fig. 2 is a schematic view of a blood purification unit 14.
Fig. 3 is a diagram showing the general configuration of a blood purification system 40.
Fig. 4 is a schematic view showing an example of the data structure of a result table 553.
Fig. 5 is a diagram of the general configuration of a server 80.
Fig. 6 is a flow chart showing an example of operation for learning processing by a control apparatus 50.
Fig. 7 is a flow chart showing an example of operation for control processing by the control apparatus 50.
Fig. 8 is a flow chart showing an example of operation for learning processing by the server 80.
Fig. 9 is a schematic view of another blood purification unit 14-2.
Fig. 10 is a schematic view of another blood purification unit 14-3.

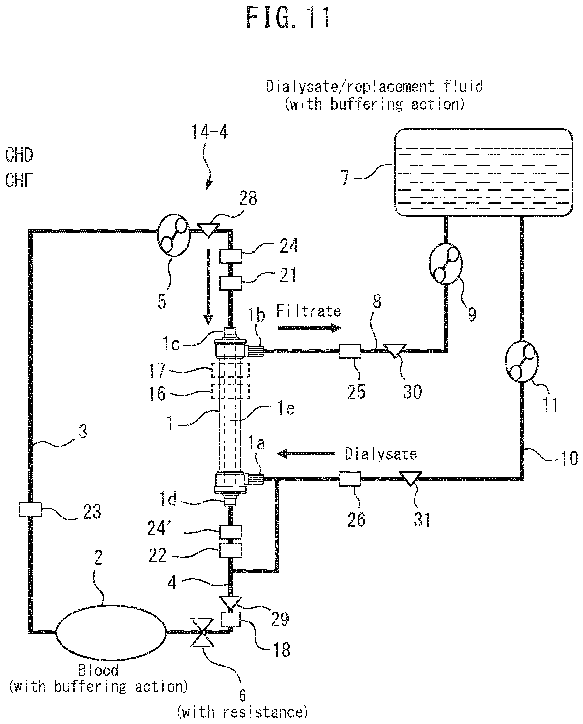

Fig. 11 is a schematic view of another blood purification unit 14-4.

DESCRIPTION OF EMBODIMENTS

Hereinafter, a blood purification system, a control method, a control program, a learning apparatus and a learning method according to an embodiment, will be described with reference to the drawings. However, it should be noted that the technical scope of the invention is not limited to these embodiments, and extends to the inventions described in the claims and their equivalents.

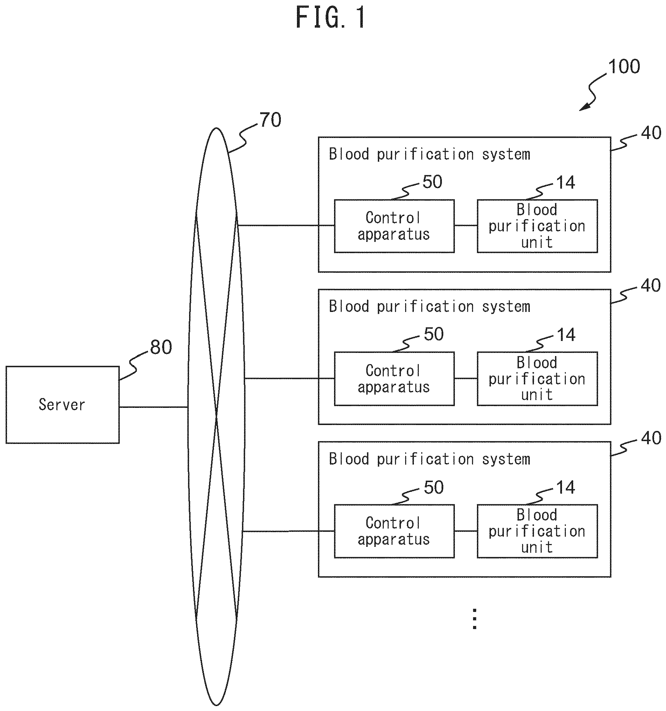

Fig. 1 is a diagram showing the general configuration of a management system 100 according to an embodiment.

As shown in Fig. 1, the management system 100 has one or more blood purification systems 40 and a server 80. Each blood purification system 40 is connected with the server 80 in a mutually communicable manner via a network 70. Each blood purification system 40 is an extracorporeal circulation blood purification system. Each blood purification system 40 carries out continuous hemodialysis filtration (CHDF), continuous hemofiltration (CHF), continuous hemodialysis (CHD) or apheresis. Each blood purification system 40 has a blood purification unit 14 and a control apparatus 50. The network 70 is a wired network such as the internet or an intranet. The network 70 may also be a wireless network such as a wireless LAN (Local Area Network).

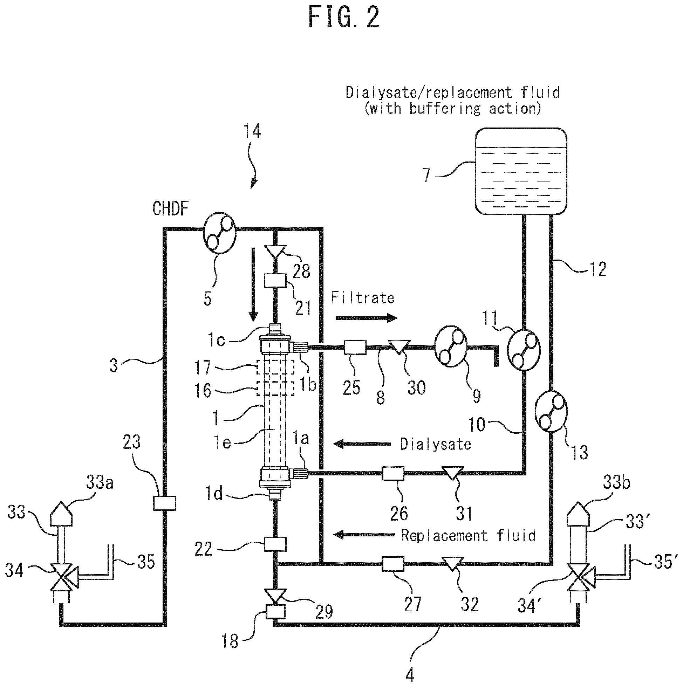

Fig. 2 is a schematic view of a blood purification unit 14 in the blood purification system 40.

As shown in Fig. 2, the blood purification unit 14 has a blood purification device 1, a blood supply line 3, a blood return line 4, a blood pump 5, a replacement fluid container 7, a filtrate line 8, a filtration pump 9, a dialysate line 10, a dialysate pump 11, a replacement fluid line 12, a replacement fluid pump 13, a magnetic regulator 16, a blood flow detector 17, a hematocrit detector 18, first pressure gauges 21 to 23, a second pressure gauge 25, a third pressure gauge 26, a fourth pressure gauge 27, first flow meters 28, 29, a second flow meter 30, a third flow meter 31, a fourth flow meter 32, tubing systems 33, 33', (three-way) valves 34, 34' and tubing systems 35, 35'. The blood purification unit 14 is intended for use in a blood purification device 1 in a clinical setting. The blood purification unit 14 carries out continuous hemodialysis filtration, continuous hemofiltration, continuous hemodialysis or apheresis.

The blood supply line 3, blood return line 4, filtrate line 8, dialysate line 10, replacement fluid line 12 and tubing systems 33, 33' are examples of lines through which liquids containing blood or filtrate flow, as a blood circuit. Polyvinyl chloride tubes, for example, are used for the blood supply line 3, blood return line 4, filtrate line 8, dialysate line 10, replacement fluid line 12 and tubing systems 33, 33'. The blood supply line 3 and blood return line 4 use tubes with lengths and diameters such that the total volume of internally contained liquid is approximately 150 ml, for example. Other members may also be used for the blood supply line 3, blood return line 4, filtrate line 8, dialysate line 10, replacement fluid line 12 and tubing systems 33, 33'.

The tubing system 33 is a blood circuit and has a blood collector 33a for collection of blood from a patient (animal or human). The tubing system 33' is a blood circuit and has a blood return unit 33b directed toward the patient. The blood supply line 3 sends blood removed from the blood collector 33a to the blood purification device 1 through the blood pump 5. The blood return line 4 sends blood flowing out from the blood purification device 1 to the blood return unit 33b. The filtrate line 8 is connected to the dialysate outlet 1b of the blood purification device 1, and sends liquid, replacement fluid and/or dialysate containing waste products, flowing out from the dialysate outlet 1b, as filtrate to the outside via the filtration pump 9. The dialysate line 10 is connected to the replacement fluid container 7 and the dialysate inlet 1a of the blood purification device 1, and it sends dialysate that has flowed out from the replacement fluid container 7 to the dialysate inlet 1a via the dialysate pump 11. The replacement fluid line 12 is connected to the replacement fluid container 7, blood supply line 3 and/or blood return line 4, and it sends replacement fluid that has flowed out from the replacement fluid container 7, to the blood supply line 3 and/or blood return line 4 via the replacement fluid pump 13. Transfer of replacement fluid to the blood supply line 3 and/or blood return line 4 via the replacement fluid pump 13 can be accomplished using a three-way valve (not shown), for example.

The blood purification device 1 purifies blood flowing through each line of the blood purification unit 14. The blood purification device 1 is a blood filter that carries out continuous slow blood filtration. The blood purification device 1 may also be a hemodialyzer (dialyzer). The blood purification device 1 separates waste products from blood during filtration or dialysis of the blood. The blood purification device 1 comprises a dialysate inlet 1a, a dialysate outlet 1b, an inlet port 1c, an outlet port 1d and a hollow membrane 1e. The inlet port 1c is an inlet for blood removed from the patient and/or replacement fluid supplied from the replacement fluid container 7 containing and/or fluid. The hollow membrane 1e is composed of a bundle of numerous hollow fiber membranes through which blood and/or replacement fluid that has flowed in through the blood inlet port 1c passes. The outlet port 1d is an outlet for blood and/or replacement fluid passing through the hollow membrane 1e to the outside. The dialysate inlet 1a is an inlet for dialysate to the hollow membrane 1e. The dialysate outlet 1b is a purification port, being a discharge port for liquid and/or replacement fluid containing waste product that has passed to the outer side of the hollow membrane 1e, and/or an outlet for dialysate passing to the outer side of the hollow membrane 1e. The blood purification device 1 used may be a common publicly known blood purification device.

The replacement fluid container 7 supplies dialysate to the blood purification device 1 by supplying the dialysate to the dialysate line 10, and/or supplies replacement fluid to the blood supply line 3 and blood return line 4 by supplying the replacement fluid to the fluid replacement line 12. The replacement fluid container 7 used may be a common publicly known replacement fluid container. The blood purification unit 14, provided with the replacement fluid container 7, can use the blood purification device 1 to carry out filtration and/or removal of water from liquid (blood) in the line. When the liquid volume in the replacement fluid container 7 is less than 100 ml, the liquid volume in the replacement fluid container 7 may be insufficient during operation of the blood purification unit 14, potentially hampering circulation of the liquid, and therefore the liquid volume in the replacement fluid container 7 is preferably 100 ml or greater. A dialysate supply device allowing constant supply of the dialysate and/or replacement fluid may also be used instead of the replacement fluid container 7. The replacement fluid container 7 and dialysate supply device are examples of supply devices.

The blood pump 5 is provided in the blood supply line 3 and controls the flow of blood in the blood supply line 3. The filtration pump 9 is provided in the filtrate line 8 and controls the flow of filtrate in the filtrate line 8. The dialysate pump 11 is provided in the dialysate line 10 and controls the flow of dialysate in the dialysate line 10. The fluid replacement pump 13 is provided in the fluid replacement line 12 and controls the flow of replacement fluid in the fluid replacement line 12. The blood pump 5, filtration pump 9, dialysate pump 11 and fluid replacement pump 13 are provided in a manner allowing variation in their respective drive (output) amount based on control by the control apparatus 50. A roller pump, for example, may be used for the blood pump 5, filtration pump 9, dialysate pump 11 and fluid replacement pump 13. Another publicly known pump may also be used for the blood pump 5, filtration pump 9, dialysate pump 11 and fluid replacement pump 13.

The magnetic regulator 16 is provided in a manner surrounding a predetermined location of the blood purification device 1, and it applies a magnetic field on the blood in the blood purification device 1 in a predetermined direction. The magnetic regulator 16 may be provided in a manner surrounding the body of the patient connected to the blood supply line 3, blood return line 4 or blood purification unit 14, and it may apply a magnetic field in a predetermined direction on blood in the blood supply line 3, blood return line 4 or patient's body. The magnetic regulator 16 has a cyclic magnet and applies a one-way magnetic field in the region inside the cyclic magnet, either parallel or reverse to the direction of blood flow. The magnetic regulator 16 is provided in a manner allowing variation in the strength of the applied magnetic field based on control by the control apparatus 50. The magnetic field strength is set to be sufficient enough to reduce the blood viscosity by a predetermined amount and/or to inhibit the blood flow turbulence by a predetermined amount, at the location where the magnetic field is applied. The magnetic regulator 16 used may be an electromagnet, permanent magnet or superconducting magnet, for example.

The magnetic regulator 16 can inhibit clogging of blood cell components in the blood purification device 1 and inhibit pressure increase in the blood purification device 1 while also reducing blood flow viscosity and blood flow turbulence. The blood purification unit 14 can thus lower patient blood pressure, ameliorate hypertension and reduce incidence of cardiac murmur. A one-way magnetic field strength of less than 0.01 Tesla with the magnetic regulator 16 will produce a lower magnetic force effect on erythrocytes. A magnetic regulator 16 at greater than 100 Tesla, on the other hand, tends to be expensive and difficult to manage. The one-way magnetic field strength of the magnetic regulator 16 is therefore preferably between 0.01 and 100 Tesla, and more preferably between 1 and 10 Tesla.

The blood pump 5, filtration pump 9, dialysate pump 11, fluid replacement pump 13 and magnetic regulator 16 are examples of liquid control mechanisms to control liquid flow in each line of the blood purification unit 14.

The blood flow detector 17 is provided at a predetermined location of the blood purification device 1 and detects the direction, speed and/or speed distribution of blood flow in the blood purification device 1. The blood flow detector 17 may be provided at a predetermined location of the body of the patient connected to the blood supply line 3, blood return line 4 or blood purification unit 14, and it may detect the direction, speed and/or speed distribution of blood flow in the blood supply line 3, blood return line 4 or patient's body. The blood flow detector 17 used may be an Aixplorer Ultrasonic Image Diagnosis Device by SuperSonic Imagine Co., for example. The blood purification unit 14 can rapidly and precisely detect blood flow abnormalities using the blood flow detector 17.

The hematocrit detector 18 is provided in the blood return line 4, and it measures hematocrit in blood in the blood return line 4. The hematocrit is an index of the blood concentration, represented as the volume ratio of erythrocytes in whole blood. In order to confirm whether or not there is an abnormality in the amount of plasma permeating from the blood purification device 1, the hematocrit detector 18 is preferably provided near the outlet port 1d. In order to confirm the difference between the hematocrit in blood flowing into the blood purification unit 14 and the hematocrit in blood flowing out from the blood purification unit 14, the hematocrit detector 18 is preferably provided between the blood collector 33a and the first pressure gauge 23 in the blood supply line 3, and also between a first flow meter 29 and the blood return unit 33b in the blood return line 4. The hematocrit detector 18 used may be a StatStrip™ Hb/Hct by Nova Biomedical Co., for example.

The first pressure gauge 23 is provided between the blood collector 33a and blood pump 5 of the blood supply line 3, and it measures blood pressure in the blood supply line 3. The first pressure gauge 21 is provided between the blood pump 5 and blood purification device 1 of the blood supply line 3, and it measures blood pressure in the blood supply line 3. The first pressure gauge 22 is provided between the blood purification device 1 and blood return unit 33b of the blood return line 4, and it measures blood pressure in the blood return line 4. The first pressure gauge 21 and first pressure gauge 22 are able to constantly measure pressure input and output for the blood purification device 1, the first pressure gauge 21 and first pressure gauge 22 allowing measurement of blood pressure loss by the blood purification device 1. The second pressure gauge 25 is provided in the plasma line 8, and it measures plasma pressure in the plasma line 8. The third pressure gauge 26 is provided in the dialysate line 10, and it measures dialysate pressure in the dialysate line 10. The fourth pressure gauge 27 is provided in the fluid replacement line 12, and it measures fluid replacement pressure in the fluid replacement line 12. The first pressure gauges 21 to 23, second pressure gauge 25, third pressure gauge 26 and fourth pressure gauge 27 used may each be a known water pressure gauge such as a semiconductor piezo resistance diffusion pressure sensor, for example.

The first flow meter 28 is provided in the blood supply line 3 and measures blood flow in the blood supply line 3. The first flow meter 29 is provided in the blood return line 4 and measures blood flow in the blood return line 4. The second flow meter 30 is provided in the filtrate line 8 and measures filtrate flow in the filtrate line 8. The third flow meter 31 is provided in the dialysate line 10, and it measures dialysate flow in the dialysate line 10. The fourth flow meter 32 is provided in fluid replacement line 12, and it measures fluid replacement flow in the fluid replacement line 12. With the first flow meters 28, 29, second flow meter 30, third flow meter 31 and fourth flow meter 32, it is possible to determine whether or not the flow rate in each line, controlled with a pump in each line, is a flow rate within a set range. The first flow meters 28, 29, second flow meter 30, third flow meter 31 and fourth flow meter 32 used may be publicly known flow meters such as Coriolis mass flow meters, electromagnetic mass flow meters, or ultrasonic mass flow meters.

The blood flow detector 17, hematocrit detector 18, first pressure gauges 21 to 23, second pressure gauge 25, third pressure gauge 26, fourth pressure gauge 27, first flow meters 28, 29, second flow meter 30, third flow meter 31 and fourth flow meter 32 are examples of detectors (sensors) that detect blood information for blood flowing through each line of the blood purification unit 14.

In a clinical setting, the blood purification unit 14 may include an anticoagulant syringe, bubble detector and alarm function. For safer use, the blood purification system 40 preferably comprises an electric power generator or battery to allow operation during disasters or power outages.

Blood removed from the patient through the blood collector 33a is sent by the blood pump 5 to the blood purification device 1 through the blood supply line 3. Blood sent to the blood purification device 1 flows into the hollow membrane 1e from the inlet port 1c, and flows out from the outlet port 1d into the blood return line 4. At the same time, dialysate supplied from the replacement fluid container 7 flows through the dialysate line 10 to the blood purification device 1 by the dialysate pump 11. Dialysate sent to the blood purification device 1 flows in from the dialysate inlet 1a and passes to the outer side of the hollow membrane 1e, being dialyzed with the liquid in the hollow membrane 1e while flowing out from the dialysate outlet 1b to the filtrate line 8. Replacement fluid supplied from the replacement fluid container 7 passes through the fluid replacement line 12 and is sent to the blood supply line 3 and blood return line 4 by the fluid replacement pump 13. Replacement fluid sent to the blood supply line 3 flows into the hollow membrane 1e from the inlet port 1c of the blood purification device 1, and flows out from the dialysate outlet 1b to the filtrate line 8 together with liquid containing waste product. Dialysate, replacement fluid and waste product-containing liquid that have flowed out into the filtrate line 8 are discharged as filtrate out of the unit by the filtration pump 9. The blood and replacement fluid that have flowed out to the blood return line 4 are returned to the patient.

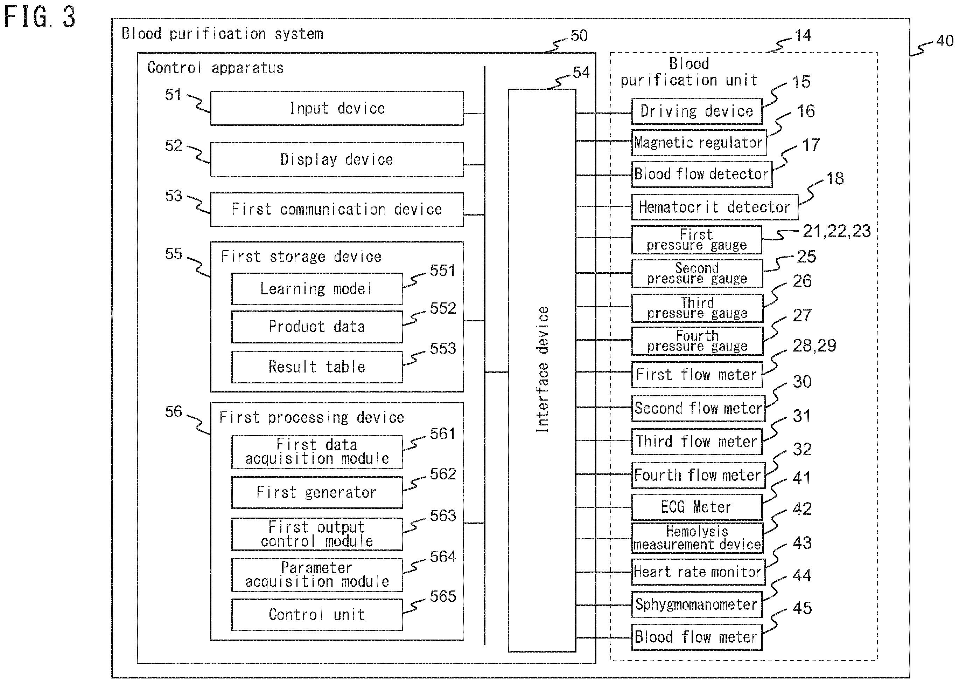

Fig. 3 is a diagram showing the general configuration of a blood purification system 40.

The blood purification unit 14 of the blood purification system 40 also has a driving device 15, ECG meter 41, hemolysis measurement device 42, heart rate monitor 43, sphygmomanometer 44 and blood flow meter 45, in addition to the configuration described above.

The driving device 15 includes one or more motors and drives the blood pump 5, filtration pump 9, dialysate pump 11 and replacement fluid pump 13 based on a control signal from the control apparatus 50, controlling flow of liquid in each line of the blood purification unit 14.

The ECG meter 41 is mounted on a patient connected to the blood purification unit 14, the patient electrocardiogram (heart rate waveform) is measured, and the measured electrocardiogram is outputted. The ECG meter 41 used may be a wearable ECG meter such as an Apple Watch (Series 4, 5, 6), for example. An Apple Watch (Series 4, 5, 6) has a crystal and electrodes, and it coordinates with an electrocardiogram application to record an electrocardiogram, similar to a type I induction electrocardiogram. The ECG meter 41 constantly detects heart rhythm and gives a notification when it has detected an irregular heart rhythm, as a sign of atrial fibrillation (AFib).

The hemolysis measurement device 42 is connected to a patient connected to the blood purification unit 14 and measures the degree of hemolysis of the patient. Hemolysis is destruction of blood cells, specifically erythrocytes. The hemolysis measurement device 42 used may be a blood leak detector incorporated into any commercially available dialysis monitoring device (such as a TR-3300 M by Toray Co., Ltd.).

The heart rate monitor 43 and sphygmomanometer 44 are mounted on patients connected to the blood purification unit 14, measuring the patient pulse and blood pressure. The blood flow meter 45 is connected to a patient connected to the blood purification unit 14 and measures the circulating blood flow of the patient. The heart rate monitor 43, sphygmomanometer 44 and blood flow meter 45 may be publicly known measuring devices.

The control apparatus 50 is an example of a learning apparatus and may be an information processing device such as a personal computer. The control apparatus 50 has an input device 51, display device 52, first communication device 53, interface device 54, first storage device 55 and first processing device 56. The input device 51, display device 52, first communication device 53, interface device 54, first storage device 55 and first processing device 56 are mutually connected via a CPU (Central Processing Unit) bus.

The input device 51 has an input device such as a touch panel-type input device or a keyboard or a mouse and an interface circuit that acquires a signal from the input device, and it outputs an operation signal in response to input from the user.

The display device 52 has a display including a liquid crystal or organic EL (ElectroLuminescent) display, and an interface circuit that outputs image data onto the display to display the image data.

The first communication device 53 is an example of a communication device. The first communication device 53 has a wired communication interface circuit following a communication protocol such as TCP/IP (Transmission Control Protocol/Internet Protocol). The first communication device 53 is connected in a communicable manner with a network 70 conforming to a communication standard such as Ethernet™. The first communication device 53 sends data received from the server 80 to the first processing device 56 via the network 70, and transmits data received from the first processing device 56 through the network 70 to the server 80. The first communication device 53 may have an antenna that sends and receives wireless signals and a wireless communication interface circuit following a communication protocol such as wireless LAN, and it may be connected in a communicable manner with the network 70 conforming to a communication standard such as wireless LAN.

The interface device 54 has an interface circuit compatible with a serial bus such as a USB (Universal Serial Bus). The interface device 54 is connected to a driving device 15, magnetic regulator 16, blood flow detector 17, hematocrit detector 18, first pressure gauges 21 to 23, second pressure gauge 25, third pressure gauge 26, fourth pressure gauge 27, first flow meters 28, 29, second flow meter 30, third flow meter 31, fourth flow meter 32, ECG meter 41, hemolysis measurement device 42, heart rate monitor 43, sphygmomanometer 44 and blood flow meter 45 of the blood purification unit 14, being provided in a communicable manner with each device with which it is connected. The interface device 54 sends data received from each connected device to the first processing device 56, and transmits the data received from the first processing device 56 to each connected device. The interface device 54 may have an interface circuit compatible with a short range wireless communication standard such as Bluetooth™, for connection in a wireless communicable manner with each member of the blood purification unit 14.

The first storage device 55 is an example of a storage. The first storage device 55 has a memory device such as a RAM (Random Access Memory) or ROM (Read Only Memory), a fixed disk device such as a hard disk, or a portable storage device such as a flexible disk or optical disk. A computer program, database and table to be used for processing by the control apparatus 50 are stored in the first storage device 55. The computer program may be installed in the first storage device 55 from a computer readable portable storage medium, using a well-known set-up program. The portable storage medium may be a CD-ROM (Compact Disc Read Only Memory) or DVD-ROM (Digital Versatile Disc Read Only Memory), for example. The computer program may also be installed from a designated server.

A learning model 551, product data 552 and a result table 553, etc., are stored as data in the first storage device 55. The learning model 551 is a model for controlling the flow of blood in the blood purification unit 14. The learning model 551 is generated by the first processing device 56 or received from the server 80. The product data 552 are data relating to the blood purification unit 14, and they represent the filtration performance and dialysis performance of the blood purification device 1. The product data 552 is preset by the user using an input device 51. The results of blood purification for each blood purification by the blood purification system 40 are stored in the result table 553. The result table 553 will be described in detail below.

The first processing device 56 operates based on a program prestored in the first storage device 55. The first processing device 56 is a CPU, for example. The first processing device 56 used may be a DSP (digital signal processor), LSI (large scale integration), ASIC (Application Specific Integrated Circuit) or FPGA (Field-Programmable Gate Array). The first processing device 56 is connected to an input device 51, display device 52, first communication device 53, interface device 54 and first storage device 55, and controls each of the devices. The first processing device 56 generates a learning model 551 and uses the generated learning model 551 to control the flow of blood in the blood purification unit 14.

The first processing device 56 reads the computer program stored in the first storage device 55 and operates according to the read-out computer program. The first processing device 56 thus functions as a first data acquisition module 561, a first generation module 562, a first output control module 563, a parameter acquisition module 564 and a control module 565. The first data acquisition module 561, first generation module 562 and first output control module 563 are examples of a data acquisition module, a generation module and an output control module, respectively.

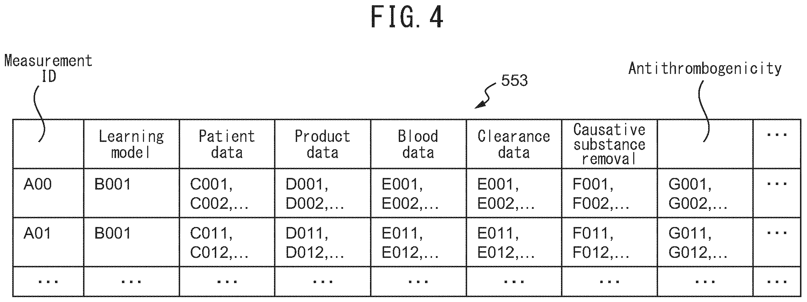

Fig. 4 is a schematic view showing an example of the data structure of a result table 553.

The relative ID No. (measurement ID), learning model, patient data, product data, blood data, clearance data, causative substance removal amount and antithrombogenicity are stored in the result table 553 for each blood purification by the blood purification system 40. The learning model is a learning model 551 used for blood purification. The identification information or storage address of the learning model 551 used for blood purification may also be stored for the learning model. Patient data is data relating to the patient including the name, body height and body weight of the patient undergoing blood purification. The product data is product data 552 that has been preset by the user.

The blood data is data relating to blood flowing into each line of the blood purification unit 14, and it includes the pulse, blood pressure, circulating blood flow, blood hematocrit or degree of hemolysis of the patient before blood purification. The clearance data is data relating to blood that has been purified by the blood purification system 40, and it includes the pulse, blood pressure, circulating blood flow, blood hematocrit or degree of hemolysis of the patient after blood purification. The clearance data may also include blood pressure, blood pressure loss, filtrate pressure, dialysate pressure, replacement fluid pressure, blood flow, filtrate flow, dialysate flow or replacement fluid flow, which are measured during blood purification. Causative substance removal is the amount of causative substances removed by the blood purification unit 14. Antithrombogenicity is the ability to control blood clotting activation, and it is the degree of thrombus formed in blood purified by the blood purification unit 14.

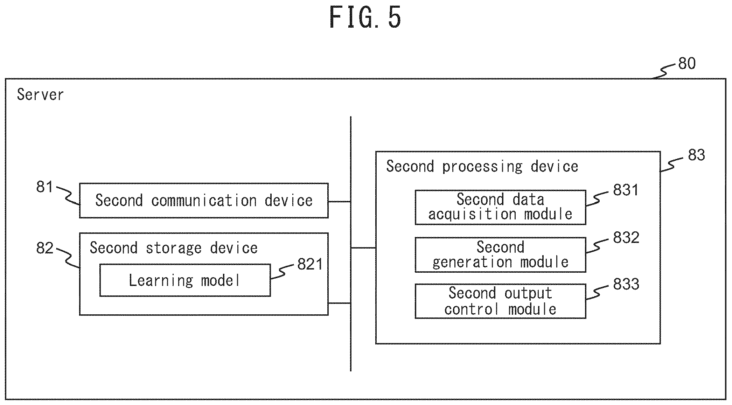

Fig. 5 is a diagram showing the general configuration of a server 80.

The server 80 is high-end computer of the control apparatus 50, and is an example of a learning apparatus. The server 80 has a second communication device 81, second storage device 82 and second processing device 83. The second communication device 81, second storage device 82 and second processing device 83 are mutually connected through a CPU bus.

The second communication device 81 is an example of a communication device for communication with multiple blood purification systems 40. The second communication device 81 has a wired communication interface circuit following a communication protocol such as TCP/IP (Transmission Control Protocol/Internet Protocol). The second communication device 81 is connected in a communicable manner with a network 70 conforming to a communication standard such as Ethernet™. The second communication device 81 sends data received from the control apparatus 50 to the second processing device 83 via the network 70, and transmits data received from the second processing device 83 through the network 70 to the control apparatus 50. The second communication device 81 may have an antenna that sends and receives wireless signals and a wireless communication interface circuit following a communication protocol such as wireless LAN, and it may be connected in a communicable manner with the network 70 conforming to a communication standard such as wireless LAN.

The second storage device 82 has a memory device such as a RAM or ROM, a fixed disk device such as a hard disk, or a portable storage device such as a flexible disk or optical disk. A computer program, database and table to be used for processing by the server 80 are stored in the second storage device 82. The computer program may be installed in the second storage device 82 from a computer readable portable storage medium, using a well-known set-up program. The portable storage medium may be a CD-ROM or DVD-ROM, for example. The computer program may also be installed from a designated server.

A learning model 821, etc., are stored as data in the second storage device 82. The learning model 821 is generated by the second processing device 83 or received from the control apparatus 50.

The second processing device 83 operates based on a program prestored in the second storage device 82. The second processing device 83 is a CPU, for example. The second processing device 83 used may be a DSP, LSI, ASIC or FPGA. The second processing device 83 is connected to a second communication device 81 and second storage device 82, and controls each of the devices. The second communication device 81 generates a learning model 821 and sends it to each control apparatus 50.

The second processing device 83 reads the computer program stored in the second storage device 82 and operates according to the read-out computer program. The second processing device 83 thus functions as a second data acquisition module 831, a second generation module 832 and a second output control module 833.

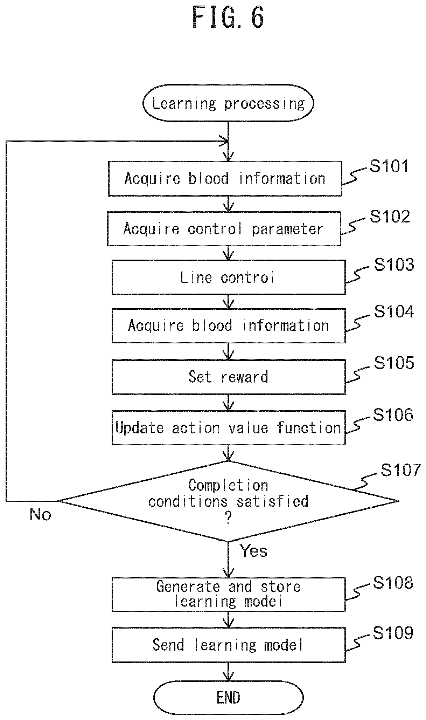

Fig. 6 is a flow chart showing an example of operation for learning processing by a control apparatus 50.

An example of operation for learning processing by the control apparatus 50 will now be explained with reference to the flow chart in Fig. 6. The flow of operation described below is carried out in cooperation with each of the elements of the control apparatus 50 primarily by the first processing device 56, based on a program prestored in the first storage device 55.

First, the first data acquisition module 561 acquires blood information relating to blood flowing through each line of the blood purification unit 14 (step S101). The blood information includes the degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, or degree of hemolysis, pulse, blood pressure or circulating blood flow of patient. The blood information includes one or more of the aforementioned parameters.

The first data acquisition module 561 acquires the direction of blood flow in the blood purification unit 14 and its speed and/or speed distribution from the blood flow detector 17 via the interface device 54, and calculates the degree of blood flow turbulence and blood vorticity based on the acquired information. For example, the first data acquisition module 561 calculates the Reynolds number for each blood flow detected by the blood flow detector 17, and determines whether each blood flow is turbulent or laminar based on whether or not the calculated Reynolds number is equal to or more than a predetermined threshold value. The first data acquisition module 561 calculates the degree of turbulent flow as the ratio of the number of turbulent blood flows with respect to the total number of blood flows detected by the blood flow detector 17. The first data acquisition module 561 also calculates the speed vector of blood flow based on the direction and speed of blood flow acquired from the blood flow detector 17, and calculates the vorticity as the rotation of the vector field formed by the calculated speed vector. Vorticity is the state of rotating flow expressed as a quantity.

The first data acquisition module 561 acquires an electrocardiogram of the patient connected to the blood purification unit 14 from the ECG meter 41 via the interface device 54, and calculates the degree of cardiac murmur based on the acquired electrocardiogram. Cardiac murmur is a disturbed temperament of heart noise out of harmony with the heart beat. Heart noise is a characteristic abnormal sound produced when blood flows through a cardiac valve or blood vessel near the heart (valve opening or closing). Abnormal sound is mainly produced due to a faulty cardiac valve. The first data acquisition module 561 calculates the degree of cardiac murmur as the occurrence rate of irregular heart rhythm, suggesting atrial fibrillation, in a heart rhythm represented in an acquired electrocardiogram. For example, the control apparatus 50 prestores in the first storage device 55 a waveform pattern for heart rhythm of atrial fibrillation. The first data acquisition module 561 uses publicly known pattern matching technology to detect waveforms similar to the heart rhythm for atrial fibrillation within a heart rhythm represented in the acquired electrocardiogram. The first data acquisition module 561 may also detect the P wave corresponding to each R wave in the heart rhythm represented in the acquired electrocardiogram, and based on the number of disappeared P waves, may detect occurrence of heart rhythms suggesting atrial fibrillation.

The first data acquisition module 561 also acquires blood pressure from first pressure gauges 21 to 23 via the interface device 54. The first data acquisition module 561 also calculates blood pressure loss as the value of the blood pressure acquired from the first pressure gauge 21 minus the blood pressure acquired from the first pressure gauge 22. The first data acquisition module 561 also acquires filtrate pressure, dialysate pressure and replacement fluid pressure from the second pressure gauge 25, third pressure gauge 26 and fourth pressure gauge 27, respectively, via the interface device 54. The first data acquisition module 561 also acquires blood flow, filtrate flow, dialysate flow and replacement fluid flow from the first flow meters 28, 29, second flow meter 30, third flow meter 31 and fourth flow meter 32, respectively, via the interface device 54.

The first data acquisition module 561 also calculates filtrate volume of the blood purification device 1 per unit time from the start of operation of the blood purification unit 14 to the current time, based on the filtrate flow acquired from the second flow meter 30. The first data acquisition module 561 also calculates the current pressure difference between the filtrate pressure acquired from the second pressure gauge 25 and the blood pressure acquired from the first pressure gauge 21. The first data acquisition module 561 further calculates fouling of the blood purification device 1, as the value calculated for the filtrate volume divided by the calculated pressure difference, and further divided by unit time.

The first data acquisition module 561 acquires the blood hematocrit value from the hematocrit detector 18, via the interface device 54. The first data acquisition module 561 further acquires the degree of hemolysis, pulse, blood pressure and circulating blood flow of the patient connected to the blood purification unit 14, from the hemolysis measurement device 42, heart rate monitor 43, sphygmomanometer 44 and blood flow meter 45, respectively, via the interface device 54.

The first data acquisition module 561 then acquires control parameters relating to the flow of liquid in the blood purification unit 14 (step S102). The control parameters include the magnetic field strength applied to the blood by the magnetic regulator 16 in a predetermined direction, or the drive amount of the blood pump 5, filtration pump 9, dialysate pump 11 or fluid replacement pump 13. The control parameters include one or more of the aforementioned parameters.

For example, the control apparatus 50 stores various usable values in the first storage device 55 for each control parameter type, and the first data acquisition module 561 acquires the control parameters by successively reading the values stored in the first storage device 55. The first data acquisition module 561 may also acquire control parameters by generating a random number value in usable ranges for each of the control parameter types. The control apparatus 50 may also store optimum values for the different control parameters in a first storage device 55 for each of the control parameter types, and the first data acquisition module 561 may acquire control parameters with fine adjustment of the optimum values.

The first data acquisition module 561 then controls the magnetic regulator 16, or controls the blood pump 5, filtration pump 9, dialysate pump 11 or replacement fluid pump 13 via the driving device 15, based on the acquired control parameters (step S103).

The first data acquisition module 561 subsequently acquires blood information in the same manner as for the processing in step S101 (step S104). The first data acquisition module 561 thus controls the liquid control mechanisms of the blood purification unit 14 based on the control parameters acquired in step S102, and acquires blood information detected by the detectors before and after control of the liquid control mechanisms, based on the control parameters. The first data acquisition module 561 thereby acquires sets of blood information and control parameters.

The first generation module 562 next sets a reward for the learning model 551 based on the blood information acquired by the first data acquisition module 561 (step S105).

The learning model 551 used by the blood purification system 40 is learned by reinforcement learning, for example. Reinforcement learning by the learning model 551 may be Q-learning, for example. The learning model 551 has an action value function that determines the value of control based on different control parameters, with the blood purification unit 14 as the environment, the control apparatus 50 as the agent, blood information as the condition and control of the liquid control mechanism based on the control parameter as the action. Each item of blood information is a physical quantity that varies with change in each control parameter. That is, a correlation exists between each item of blood information and each control parameter, and the first generation module 562 can generate the learning model 551 allowing suitable control parameters to be set, corresponding to the state of the blood purification unit 14.

With Q-learning, the environment state "s" and the action "a" selected in that state "s" are used as independent variables for learning of the action value function Q(s, a), which represents the value of action with selection of action "a" at state "s". In Q-learning, learning begins in a state with unknown correlation between state "s" and action "a", and selection of different action "a" values for a given state "s" is repeated by trial and error for repeated update of the action value function Q. The configuration for Q-learning is such that a reward r is obtained when an action "a" has been selected for a certain state "s", the action value function Q being learned so as to select actions "a" that give higher rewards r. Updating of the action value function Q is represented by the following formula.

[Mathematical Formula 1]

$$Q(s_t, a_t) \leftarrow Q(s_t, a_t) + \alpha(r_{t+1} + \gamma \max_a Q(s_{t+1}, a) - Q(s_t, a_t))$$

In this formula, $s_t$ and $a_t$ are, respectively, the state and action at each time t, the state with action $a_t$ varying from $s_t$ to $s_{t+1}$. The value of $r_{t+1}$ is the reward obtained when the state changes from $s_t$ to $s_{t+1}$. The variable maxQ is the value of Q when carrying out action "a" which gives the maximum value of Q at time t + 1 (as assumed at time t). The variables $\alpha$ and $\gamma$ are the learning coefficient and discount factor, respectively, which are arbitrarily set to $0 < \alpha \leq 1$

(normally 0.9 to 0.99) and $0 < \gamma \leq 1$ (normally about 0.1).

This update formula indicates that $Q(s_t, a_t)$ is increased if, compared to the evaluation value $Q(s_t, a_t)$ for action $a_t$ at a given state $s_t$, the evaluation value $Q(s_{t+1}, maxa_{t+1})$ for the optimal action $maxa_{t+1}$ at the next state $s_{t+1}$ is greater, and $Q(s_t, a_t)$ is decreased if it is smaller. In other words, with this update formula the value of a given action in a given state can approach the value of the optimal action at the next step. The action value function is therefore updated so as to increase the action value for action (operation conditions) to create the most optimal state for operation of the blood purification unit 14, i.e. to increase the action value of the optimal action (operation conditions) of the blood purification unit 14.

The initial value for each action value function Q is arbitrarily set. The first generation module 562 uses the number of executions of step S106 as the time in the formula. The first generation module 562 identifies the blood information before control acquired in step S101 as state $s_t$, specifies control based on the control parameter acquired in step S102 as action $a_t$, and specifies the blood information after control acquired in step S104 as state $s_{t+1}$.

The first generation module 562 sets a reward r based on the change in blood information (state) before and after control. The first generation module 562 sets the reward r so that the reward r is greater as change (difference) in the blood information is smaller after control with respect to the blood information before control, and the reward r is smaller as change (difference) is larger. For example, setting one or more thresholds in advance for each blood information item, the first generation module 562 sets a reward r by comparing the amount of change in each blood information item with the respective threshold. When the amount of change is greater than the threshold maximum, the first generation module 562 may set the reward r to be 0. The first generation module 562 thus sets the reward r so that, when the blood purification unit 14 has been controlled based on a specific control parameter, the reward r is higher with a more stable state of the blood in the blood purification unit 14. This allows the first generation module 562 to generate a learning model 551 trained so as to select a control parameter that results in a more stable blood state in the blood purification unit 14 with respect to the current blood state.

The first generation module 562 then updates the action value function based on the sets of the blood information acquired by the first data acquisition module 561 and the control parameters, and the set reward (step S106). The first generation module 562 updates the action value function $Q(s_t, a_t)$ according to the aforementioned formula based on the specific state $s_t$, action $a_t$, state $s_{t+1}$ and set reward r.

The first generation module 562 then determines whether or not the conditions for completion of learning have been satisfied (step S107). The conditions for completion of learning are set beforehand, as being when the total number of updates of the action value function is equal to or more than a predetermined number, or when the maximum or minimum number of updates for each action value function is equal to or more than a predetermined number. When the conditions for completion of learning have not been satisfied, the first data acquisition module 561 and first generation module 562 return to the processing of step S101 and repeat the processing of steps S101 to S107. The first data acquisition module 561 thus acquires a plurality of sets of blood information and control parameters, and the first generation module 562 uses each set acquired by the first data acquisition module 561 to update the action value function. From the second processing onward, the processing in step S101 may be omitted and the first data acquisition module 561 may use the blood information after control that was previously acquired in step S104, as the blood information before control.

When the conditions for completion of learning have been satisfied, the first generation module 562 generates a learning model 551 having sets (an action value table) of each of the finally updated Q(s, a) values (action values), and stores it in the first storage device 55 (step S108). The learning model 551 is trained so that when predetermined blood information is input as the state, it outputs control parameters corresponding to action with the highest action value in that state, i.e. control parameters that allow maximum stabilization of the blood information. The learning model 551 may also be trained so that when predetermined blood information is input as the state, it outputs the action value for each control parameter (each value) in that state.

This allows the blood purification system 40 to automatically create the optimal operation conditions for each time period elapsed after beginning operation of the blood purification unit 14. As a result, the blood purification system 40 can derive the optimal operation conditions for continuous hemodialysis filtration, continuous hemofiltration, continuous hemodialysis and apheresis, for example.

The first output control module 563 then outputs the learning model 551 generated by the first generation module 562 by sending it to the server 80 via the first communication device 53 (step S109), and the series of steps is complete. The learning model 551 is an example of information relating to the learning model. After the learning model 551 has been received from the control apparatus 50 via the second communication device 81, the server 80 stores the received learning model 551 in the second storage device 82 as a learning model 821, while also sending it to the other control apparatus 50 via the second communication device 81. After the learning model 821 has been received from the server 80 via the first communication device 53, the other control apparatus 50 stores the received learning model 821 in the first storage device 55 as a learning model 551. The management system 100 can share the learning model among multiple blood purification systems 40, thereby increasing generation efficiency by the learning model. The processing of step S109 can be omitted, and the control apparatus 50 may use the learning model 551 generated by its own device only in its own device.

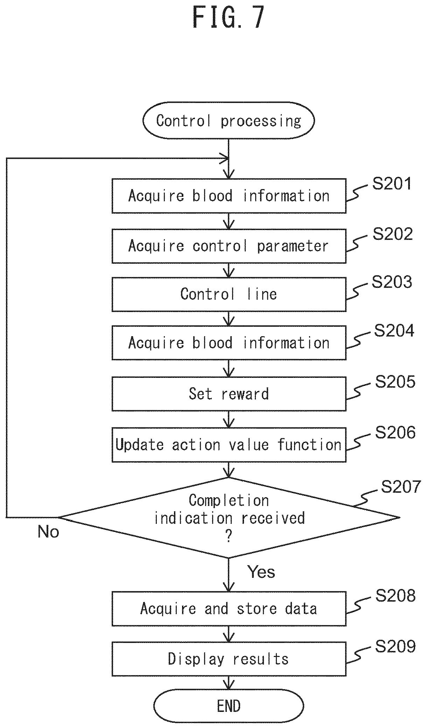

Fig. 7 is a flow chart showing an example of operation for control processing by the control apparatus 50.

An example of operation for control processing by the control apparatus 50 will now be explained with reference to the flow chart in Fig. 7. The flow of operation described below is carried out in cooperation with each of the elements of the control apparatus 50 primarily by the first processing device 56, based on a program prestored in the first storage device 55. Control processing is carried out when the user has used the input device 51 to indicate that blood purification is to begin.

First, the parameter acquisition module 564 acquires from the blood purification unit 14 the blood information detected by the detector of the blood purification unit 14, in the same manner as treatment in step S101 of Fig. 6, and stores it in the first storage device 55 (step S201).

The parameter acquisition module 564 then inputs the acquired blood information into the learning model 551 stored in the first storage device 55 and acquires the control parameter output from the learning model 551 (step S202).

The control module 565 then controls the magnetic regulator 16, or controls the blood pump 5, filtration pump 9, dialysate pump 11 or replacement fluid pump 13 via the driving device 15, based on the control parameters acquired by the parameter acquisition module 564 (step S203).

The first data acquisition module 561 subsequently acquires blood information in the same manner as for the processing in step S 104 of Fig. 6, and stores it in the first storage device 55 (step S204).

Next, the first generation module 562 determines a reward for the learning model 551 in the same manner as for the processing in step S105 of Fig. 6 (step S205). The first generation module 562 sets the reward based on the change in the blood information acquired in step S201 and the blood information acquired in step S204.

Next, the first generation module 562 updates the action value function in the same manner as for the processing in step S106 of Fig. 6 (step S206). The first generation module 562 updates the action value function based on the blood information acquired in step S201, the blood information acquired in step S204, the control parameter acquired in step S202 and the reward set in step S206. The processing of steps S204 to S206 may be omitted, and the first generation module 562 does not need to update the learning model 551 in the control processing.

The control module 565 then determines whether or not the user has used the input device 51 to indicate that the blood purification is complete (step S207). If it has not yet been indicated that blood purification is complete, the control module 565 returns to the processing of step S201 and repeats the processing of steps S201 to S207.

If it has been indicated that blood purification is complete, the control module 565 stores the results of the control processing in the result table 553 (step S208).

The control module 565 reads out from the first storage device 55 the learning model 551 used for the current blood purification. The control module 565 also receives the input of patient data by the user using the input device 51. The control module 565 further reads out the product data 552 of the blood purification device 1 and replacement fluid container 7 from the first storage device 55. The control module 565 further reads out from the first storage device 55 the blood information that was initially stored in step S201, and acquires it as blood data relating to blood flowing through each line of the blood purification unit 14. The control module 565 further reads out from the first storage device 55 each blood information item that has been stored in step S204, and acquires it as clearance data for the blood purification system 40. The control module 565 may also read out only the blood information that is finally stored in step S204 as the clearance data for the blood purification system 40.

The control module 565 also receives the input of causative substance removal from the patient, input by the user using the input device 51. A meter for measurement of causative substance removal may also be connected to the patient who is connected to the blood purification unit 14, and the control module 565 may acquire the causative substance removal information for the patient from the meter via an interface 50.

The control module 565 also receives the input of patient antithrombogenicity by the user using the input device 51. For example, after blood purification is complete and once the blood has been discharged from the blood purification unit 14, the fixing solution including glutaraldehyde for fixing of thrombi formed in the hollow membrane 1e is recirculated for a certain time in the blood supply line 3 and blood return line 4. The blood purification device 1 is then removed from the blood purification unit 14, and the hollow membrane 1e at the inlet of the removed blood purification device 1 is visually observed to evaluate the presence of thrombus formation. The state of thrombi inside the hollow membrane 1e at a predetermined site is observed using a scanning electron microscope. For example, the hollow membrane 1e is divided into the 3 regions of filtration side, center and outside (the side opposite the filtration side) in the longitudinal direction and divided into the 3 regions of inlet, center and outlet in the transverse direction, and the following evaluation is made for each 3 × 3 region.

For example, about 15 hollow fiber membranes are randomly extracted per site, the cross-sectional area of thrombus-formed sections is calculated for each hollow fiber membrane, and the average value for cross-sectional area of the thrombus-formed sections per hollow fiber membrane is calculated for each site. The ratio of the average cross-sectional area of thrombus-formed sections with respect to the average cross-sectional area of the hollow fiber membranes is calculated as the thrombus formation rate, for each site. The thrombus formation rate is used as an index for evaluation of the antithrombogenicity of the blood purification device 1. For example, the thrombus formation rate is divided into 5 levels and assigned points (score), for each site. Zero points are assigned if the thrombus formation rate is less than 1%, 1 point is assigned if the thrombus formation rate is ≥1% and <25%, 2 points are assigned if the thrombus formation rate is ≥25% and <50%, 3 points are assigned if the thrombus formation rate is ≥51% and <75%, and 4 points are assigned if the thrombus formation rate is ≥76%. The average point score may also be calculated for each region in the longitudinal direction or for each region in the transverse direction. This allows the direction of thrombus formation in the hollow membrane 1e to be determined for different regions. The average number of points at all of the sites may also be used as the evaluation score for antithrombogenicity for the blood purification device 1 as a whole. A lower evaluation score is evaluated as higher antithrombogenicity.

The control module 565 mutually associates the learning model 551, patient data, product data 552, blood data, clearance data, causative substance removal and antithrombogenicity, assigning new measurement IDs and storing them in the result table 553.

The first output control module 563 then outputs the results of blood purification by displaying them on the display device 52 (step S208), and the series of steps is complete. The results of blood purification are an example of information relating to the learning model. The user can thereby confirm the effect of the learning model 551, so that the learning model 551 exhibiting a high effect can be identified and shared with another control apparatus 50.

The control apparatus 50 may also use a learning model stored in the server 80 to acquire the control parameter, instead of using the learning model stored in its own device. In this case the parameter acquisition module 564 sends blood information to the server 80 via the first communication device 53 in step S202. The second processing device 83 of the server 80 receives blood information from the control apparatus 50 via the second communication device 81, inputs it into the learning model 821 stored in the second storage device 82, and acquires the control parameters outputted from the learning model 821. The second processing device 83 sends the acquired control parameters to the control apparatus 50 via the second communication device 81, and the parameter acquisition module 564 acquires the control parameters by receiving them from the server 80 via the first communication device 53.

The control apparatus 50 may use the learning model 821 stored in the server 80 to properly control the blood purification system 40 using the latest learning model 821 as updated by the server 80. The control apparatus 50 may also use the learning model 551 stored in its own device to properly control the blood purification system 40 even when communicable connection with the server 80 has been severed.

As described in detail above, the blood purification system 40 generates a learning model 551 based on blood information relating to blood flowing through the blood purification unit 14 and the control parameters of the blood purification unit 14, and uses the generated learning model 551 to control the blood purification unit 14. This allows the blood purification system 40 to learn, analyze and provide optimal operation conditions by itself. The blood purification system 40 can therefore better stabilize the state of blood flowing in the blood purification unit 14, and can achieve more efficient purification of blood.

Specifically, blood information includes the degree of blood flow turbulence or the blood vorticity. The present inventors have found that blood flow turbulence (a fluid with irregular movement, being in a constantly varying disturbed state) and blood vorticity promote platelet production. With large change in the degree of blood flow turbulence and blood vorticity, fouling of the blood purification device 1 tends to be accelerated. Because operation of the blood purification system 80 takes several hours to several days and there are limits to the extent of visual work and operation by humans, it is difficult for a human to ascertain the optimal conditions for degree of blood flow turbulence and blood vorticity. The blood purification system 40 can use machine learning to acquire the optimal conditions for the degree of blood flow turbulence and the blood vorticity, and particularly the optimal conditions that are difficult for a human to imagine, thereby can reduce fouling of the blood purification device 1.

Blood information also includes the degree of cardiac murmur. A greater degree of cardiac murmur tends to result in poorer efficiency of blood removal from the patient. For patients using *in vitro* blood treatment devices such as a blood purification device 1, studies have shown that more than half of those diagnosed with ischemic heart disease were previously thought to be without symptoms. Myocardial infarction tends to occur most commonly within the first year of using an *in vitro* blood treatment device. The blood purification system 40 can use machine learning to acquire the optimal conditions for degree of cardiac murmur, and particularly the optimal conditions that are difficult for a human to imagine, thereby can reduce the potential for ischemic heart disease in patients using the blood purification device 1. Moreover, since the blood purification system 80 has an ECG meter 41, it allows the patient to be aware of ischemic heart disease.

Control parameters also include magnetic field strength applied to blood in a predetermined direction. Since even slight changes in magnetic field strength significantly affect the flow of blood, it is difficult to properly control magnetic field strength. After the magnetic field strength has changed, the flow of blood does not change immediately but only after a certain period of time, thus making it difficult to properly control the magnetic field strength. It is difficult to obtain optimal conditions for magnetic field strength by visual work and operation by a human. The blood purification system 40 can use machine learning to acquire the optimal conditions for magnetic field strength, and particularly the optimal conditions that are difficult for a human to imagine.

As a result of much experimentation the present inventors have found that optimal operation conditions can be obtained by providing a blood purification system 40 with a self-learning function. In particular, the blood purification system 40 can analyze and provide operation conditions that can increase the antithrombogenicity of the blood purification device 1 used for continuous hemodialysis filtration, lengthen its lifetime, and reduce fouling.

While a period of about 3 days is generally required to obtain suitable operation conditions using a closed circulating test device, the blood purification system 40 can use machine learning technology to obtain suitable operation conditions within a short period of time. The control apparatus 50 can also evaluate the performance of the blood purification device 1 while conducting filtration and/or water removal with the blood purification unit 14, allowing more efficient performance evaluation of the blood purification device 1. The control apparatus 50 can also set the filtrate volume and drainage in the blood purification unit 14 and properly manage operation of all of the multiple pumps.

If the control apparatus 50 controlling the blood purification unit 14 generates a learning model 551, the blood purification system 40 can generate the learning model 551 using a simple configuration.

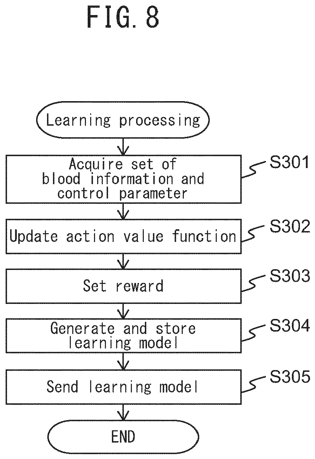

Fig. 8 is a flow chart for showing an example of operation of learning processing by the server 80 according to another embodiment. For this embodiment, the second data acquisition module 831, second generation module 832 and second output control module 833 of the server 80 are examples of the data acquisition module, generation module and output control module, respectively.

An example of operation for learning processing by the server 80 will now be explained with reference to the flow chart in Fig. 8. The flow of operation described below is carried out in cooperation with each of the elements of the server 80 primarily by the second processing device 83, based on a program prestored in the second storage device 82.

First, the second data acquisition module 831 acquires a plurality of sets of blood information and control parameters, as received from the control apparatus 50, i.e. the blood purification system 40, via the second communication device 81 (step S301). The first data acquisition module 561 of the control apparatus 50 repeatedly carries out the processing of steps S101 to S 104 in Fig. 6 to acquire a plurality of sets of blood information and control parameters, and sends them to the server 80 via the first communication device 53. The second data acquisition module 831 acquires a plurality of sets of blood information and control parameters, as received from the control apparatus 50 via the second communication device 81. Incidentally, the second data acquisition module 831 may also acquire sets of blood information and control parameters received from multiple control apparatuses 50, i.e. from multiple blood purification systems 40. This will allow the second data acquisition module 831 to more efficiently acquire learning data of the learning model 821.

Next, the second generation module 832 determines a reward corresponding to each set of blood information and control parameter, in the same manner as the processing in step S105 of Fig. 6 (step S302).

The second generation module 832 then updates the action value function for each set of blood information and control parameter, based on the set of blood information and control parameter and the set reward, in the same manner as the processing in step S106 of Fig. 6 (step S303).

The second generation module 832 then generates a learning model 821 having sets (action value table) of each of the finally updated Q(s, a) values (action values), and stores it in the second storage device 82 (step S304), in the same manner as the processing of step S108 in Fig. 6.

The second output control module 833 then outputs the learning model 821 generated by the second generation module 832 by sending it to each control apparatus 50 via the second communication device 81 (step S305), and the series of steps is complete. The learning model 821 is an example of information relating to learning model. After the learning model 821 has been received from the server 80 via the first communication device 53, each control apparatus 50 stores the received learning model 821 in the first storage device 55 as a learning model 551.

As described in detail above, the blood purification system 40 can achieve more efficient purification of blood even if the server 80 generates the learning model 821.

In particular, the management system 100 is capable of unified management of the learning model used by each blood purification system 40 at the server 80, whereby it can inhibit variation in learning model precision used by the blood purification system 40.

The learning processing represented in Fig. 8 may also be carried out by each control apparatus 50 instead of the server 80. That is, each control apparatus 50 may acquire a plurality of sets of blood information and control parameters from another control apparatus 50, and may use the acquired sets to generate a learning model 551.

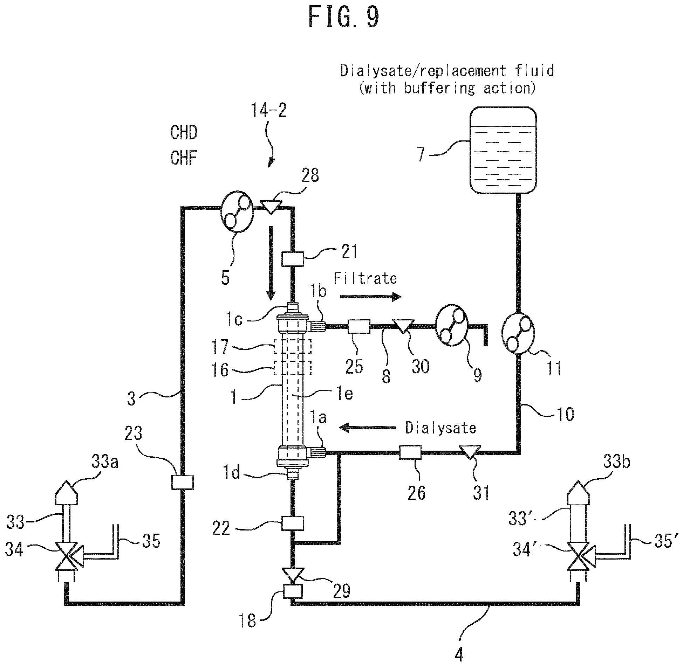

Fig. 9 is a schematic diagram showing a blood purification unit 14-2 according to another embodiment.

The blood purification unit 14-2 has each of the parts of the blood purification unit 14 and is used instead of the blood purification unit 14. However, the fluid replacement line 12, fluid replacement pump 13, fourth pressure gauge 27 and fourth flow meter 32 are omitted in the blood purification unit 14-2. The dialysate line 10 is further connected to the blood return line 4, and it sends dialysate and/or replacement fluid that has flowed out from the replacement fluid container 7, to the blood return line 4 via the dialysate pump 11. Transfer of dialysate or replacement fluid to the dialysate inlet 1a or blood return line 4 via the dialysate pump 11 can be accomplished using a three-way valve (not shown), for example.

Dialysate supplied from the replacement fluid container 7 flows through the dialysate line 10 to the blood purification device 1 by the dialysate pump 11. Dialysate sent to the blood purification device 1 flows in from the dialysate inlet 1a and passes to the outer side of the hollow membrane 1e, being dialyzed with the liquid in the hollow membrane 1e while flowing out from the dialysate outlet 1b to the filtrate line 8. Replacement fluid supplied from the replacement fluid container 7 passes through the dialysate line 10 and is sent to the blood return line 4 by the dialysate pump 11. Dialysate and waste product-containing liquid that have flowed out into the filtrate line 8 are discharged out of the unit by the filtration pump 9. The replacement fluid that has flowed out to the blood return line 4 is returned to the patient together with blood.

The control apparatus 50 controls the blood purification unit 14-2 in a manner similar to control of the blood purification unit 14. When the blood purification unit 14-2 is used, however, the drive amount of the fluid replacement pump 13 is not included in the control parameters of the blood purification unit 14-2. When the blood purification unit 14-3 is used, the replacement fluid pressure and replacement fluid flow in the blood information are measured by the third pressure gauge 26 and third flow meter 31, respectively.

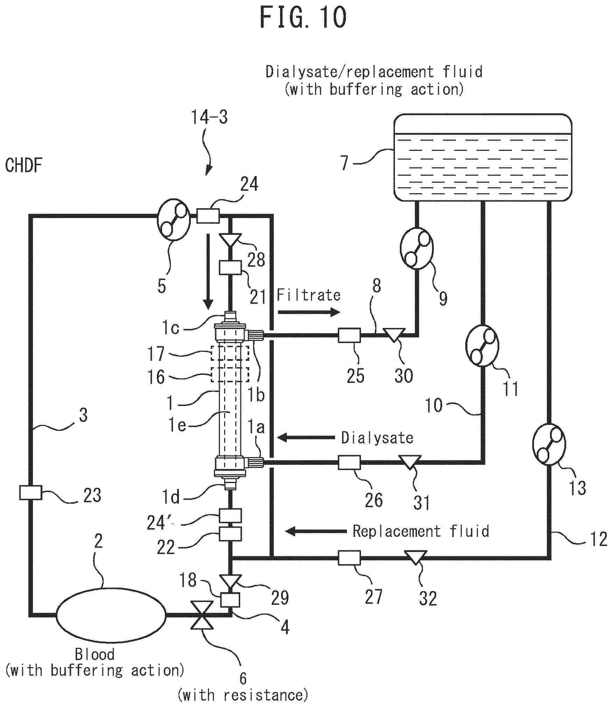

Fig. 10 is a schematic diagram showing a blood purification unit 14-3 according to another embodiment.

The blood purification unit 14-3 has each of the parts of the blood purification unit 14 and is used instead of the blood purification unit 14. However, the blood purification unit 14-3 does not have tubing systems 33, 33', (three-wave) valves 34, 34' or tubing systems 35, 35', instead having a blood bag 2, resistance members 6 and opening and closing ports 24, 24'. The filtrate line 8 is connected to the replacement fluid container 7, and it sends filtrate that has flowed out from the blood purification device 1 to the replacement fluid container 7 via the filtration pump 8. The blood purification unit 14-3 can be employed when using the blood purification device 1 for blood flow, blood pressure, filtrate volume or drainage under essentially the same conditions as for actual use, but in a non-clinical setting which is not for patients. The blood purification unit 14-3 is capable of comparative evaluation of antithrombogenicity and lifetime of the blood purification device 1, as well as evaluation of fouling.

The blood purification unit 14-3 has a closed circuit wherein test liquid flows in a circulated manner through the plasma separation device 1 without contacting the atmosphere. Human blood (whole blood) is used as the test liquid. The test liquid to be used is not limited to human blood, and the test liquid may also be another liquid such as animal blood or artificial blood with blood components similar to human blood.

The blood supply line 3 and blood return line 4 are connected via a blood bag 2.

The resistance member 6 is provided in the blood return line 4. The resistance member 6 may be provided in the blood supply line 3 either instead of or in addition to the blood return line 4. The resistance member 6 is used to apply resistance to the blood supply line 3 or blood return line 4 at their installation locations, matching the flow rate and pressure of the test liquid (blood) to the use environment. In particular, the resistance member 6 applies aperture resistance to the blood return line 4 to simulate peripheral resistance of the human body, functioning as a vein model for adjustment of flow of the test liquid to simulate a vein in the human body. The resistance member 6 uses clamps that are able to change the value of force (resistance) applied to each line by driving of a motor, for example. Various other types of devices such as valves may also be used for the resistance member 6. The resistance member 6 is an examples of liquid control mechanisms. The driving device 15 also has a motor for driving the resistance member 6.

The opening and closing ports 24, 24' are constructed with stopcocks, for example, that allow switching between an open state that permits supply or discharge of test liquid through the blood supply line 3, and a closed state which prevents supply or discharge of test liquid. The opening and closing ports 24, 24' are set in the open state when harvesting a test liquid sample during testing or when switching test liquids after testing, and are otherwise set in the closed state. When the opening and closing ports 24, 24' are set in the closed state, the circuit of the blood purification unit 14-3 as a whole is maintained in non-contact with air.

The blood purification unit 14-3 preferably comprises temperature control means to adjust the plasma separation device 1, blood bag 2, factor separating device 7 and blood purification unit 14-3, in order to maintain a constant temperature for each liquid in the blood purification unit 14-3. The temperature control means has a water tank with water stored inside it, and a heater that keeps the water temperature in the water tank at a predetermined temperature. By placing the blood bag 2 and a portion of the blood supply line 3 in the water tank, it is possible to keep the test liquid flowing through the blood supply line 3 and blood return line 4 at a constant temperature similar to human body temperature (about 36 to 37°C). Similarly, by placing the replacement fluid container 7 and portions of the filtrate line 8, dialysate line 10 and fluid replacement line 12 in the water tank, it is possible to keep the dialysate or replacement fluid flowing through the filtrate line 8, dialysate line 10 and fluid replacement line 12 at a constant temperature similar to human body temperature (about 36 to 37°C). The temperature control means may also have a heater that keeps the closed space in which the entire blood purification unit 14-2 is housed at the constant temperature.

In order to simulate the effects of differential pressure by the level difference in each device provided in the liquid circuit of the blood purification unit 14 used during actual blood filtration, each part of the blood purification unit 14-3 is disposed so as to have a similar level difference for creating differential pressure. Each part of the blood purification unit 14-3 is also disposed in a manner that also takes into account the effect of gravity by the level difference. For example, the blood pump 5 is disposed at the highest point. The blood pump 5 is disposed at a height of about 600 mm to 700 mm from the installation surface of the blood purification unit 14-3, which is the lowest point. The blood purification device 1 is held in an orientation with the inlet port 1c at the upper end and the outlet port 1d at the lower end. The top edge of the blood purification device 1 is disposed at a location at a height of about 400 mm to 500 mm from the installation surface.

The control apparatus 50 controls the blood purification unit 14-3 in a manner similar to control of the blood purification unit 14. When the blood purification unit 14-3 is used, however, the value of resistance applied by the resistance member 6 (resistance amounts) are included in the control parameters of the blood purification unit 14-3. When the blood purification unit 14-3 is used, the blood information does not include the degree of cardiac murmur, patient blood pressure, degree of hemolysis, pulse, blood pressure or circulating blood flow of patient.

By adjusting the amounts of filtrate, dialysate and/or replacement fluid in the blood purification unit 14-3, it is possible to evaluate the antithrombogenicity and/or lifetime of the blood purification device 1. A test method for evaluation of antithrombogenicity and evaluation of the lifetime of the blood purification device 1 using the blood purification unit 14-3, will now be explained.

After the blood purification device 1 to be tested has been set in the blood purification unit 14-3, the test liquid (blood) is filled into the blood supply line 3 and blood return line 4, and the dialysate or replacement fluid is filled into the filtrate line 8, dialysate line 10 and fluid replacement line 12. The blood pump 5, filtration pump 9, dialysate pump 11 and fluid replacement pump 13 are driven. Blood with a pulsatile flow created by the blood pump 5 under the same conditions as an actual use environment passes from the blood bag 2 to the blood pump 5, and flows into the blood purification device 1 from the inlet port 1c, through the hollow membrane 1e. The blood that has passed through the hollow membrane 1e flows out from the outlet port 1d to the blood return line 4. Dialysate supplied from the replacement fluid container 7 passes through the dialysate pump 11, flows in from the dialysate inlet 1a into the blood purification device 1 and passes to the outer side of the hollow membrane 1e, being dialyzed with the liquid in the hollow membrane 1e while flowing out from the dialysate outlet 1b to the filtrate line 8. Replacement fluid supplied from the replacement fluid container 7 passes through the fluid replacement pump 13 and is sent to the blood supply line 3 and blood return line 4. Replacement fluid send to the blood supply line 3 flows into the blood purification device 1 from the inlet port 1c, passes through the hollow membrane 1e, and flows out from the dialysate outlet 1b to the filtrate line 8 together with liquid containing waste product. Dialysate and waste product-containing liquid that have flowed out into the filtrate line 8 are returned to the replacement fluid container 7 by the filtration pump 9. The blood and replacement fluid that have flowed out to the blood return line 4 pass through the resistance member 6 and are returned to the blood bag 2.

The blood pump 5, filtration pump 9, dialysate pump 11 and replacement fluid pump 13 are driven continuously for a time corresponding to an actual use environment (for example, about 3 hours to 3 days). The first pressure gauges 21 to 23, second pressure gauge 25, third pressure gauge 26, fourth pressure gauge 27, first flow meters 28, 29, second flow meter 30, third flow meter 31 and fourth flow meter 32 are periodically monitored during this time, and data relating to periodic changes in the inlet pressure, outlet pressure or differential pressure of the blood purification device 1 are acquired. The control apparatus 50 can determine the lifetime of the plasma separation device 1 based on the acquired data. When the inlet pressure of the plasma separation device 1 have increased to a predetermined value (such as 150 mmHg) from the initial value (70 mmHg, for example), even if the driving time has not elapsed, the blood pump 5, filtration pump 9, dialysate pump 11 and replacement fluid pump 13 are stopped and the test is completed, and the elapsed time from the start of the test (the operating time) is also acquired as data.

During the test, blood that has flowed through the blood supply line 3 is harvested as samples at predetermined intervals, with each of the components being measured each time, and data relating to periodic changes are also acquired for each component. After the test is completed, and once the blood has been discharged from the blood purification unit 14-3, the fixing solution including glutaraldehyde for fixing of thrombi formed in the hollow membrane 1e is recirculated for a certain time in the blood supply line 3 and blood return line 4. The driving conditions for the blood pump 5, filtration pump 9, dialysate pump 11 and replacement fluid pump 13 during this time are set to be the same conditions as for testing with blood. The blood purification device 1 is also removed from the blood purification unit 14-3, and the antithrombogenicity of the blood purification device 1 is evaluated for the removed blood purification device 1. According to this embodiment, therefore, an evaluation test is carried out for the blood purification device 1 while maintaining the desired state for the flow rate and pressure and the test liquid components without contact with air, and a test that is essentially the same as under actual use conditions with a patient is carried out in a non-clinical setting without a patient.

Fig. 10 is a schematic diagram showing a blood purification unit 14-4 according to another embodiment.

The blood purification unit 14-4 has each of the parts of the blood purification unit 14-2 and is used instead of the blood purification unit 14-2. However, the blood purification unit 14-4 does not have tubing systems 33, 33', (three-wave) valves 34, 34' or tubing systems 35, 35', instead having a blood bag 2, resistance members 6 and opening and closing ports 24, 24'. The blood bag 2, resistance member 6 and opening and closing ports 24, 24' have the same construction as the blood bag 2, resistance member 6 and opening and closing ports 24, 24' of the blood purification unit 14-3. Similar to the blood purification unit 14-3, the blood purification unit 14-4 can also be employed when using the blood purification device 1 for blood flow, blood pressure, filtrate volume or drainage under essentially the same conditions as for actual use, but in a non-clinical setting which is not for patients.

The control apparatus 50 controls the blood purification unit 14-4 in a manner similar to control of the blood purification unit 14-2. When the blood purification unit 14-4 is used, however, the value of resistance applied by the resistance member 6 (resistance amounts) are included in the control parameters of the blood purification unit 14-4. When the blood purification unit 14-4 is used, the blood information does not include the degree of cardiac murmur, patient blood pressure, degree of hemolysis, pulse, blood pressure or circulating blood flow of patient.

Blood with a pulsatile flow created by the blood pump 5 in the blood purification unit 14-4 passes from the blood bag 2 to the blood pump 5, and flows into the blood purification device 1 from the inlet port 1c, through the hollow membrane 1e. The blood that has passed through the hollow membrane 1e flows out from the outlet port 1d to the blood return line 4. Dialysate supplied from the replacement fluid container 7 passes through the dialysate pump 11, flows in from the dialysate inlet 1a into the blood purification device 1 and passes to the outer side of the hollow membrane 1e, being dialyzed with the liquid in the hollow membrane 1e while flowing out from the dialysate outlet 1b to the filtrate line 8. Replacement fluid supplied from the replacement fluid container 7 passes through the dialysate line 10 and is sent to the blood return line 4 by the dialysate pump 11. Dialysate and waste product-containing liquid that have flowed out into the filtrate line 8 are returned to the replacement fluid container 7 by the filtration pump 9. The blood and replacement fluid that have flowed out to the blood return line 4 pass through the resistance member 6 and are returned to the blood bag 2.

This embodiment is not limited to the description given above. For example, the control apparatus 50 and blood purification unit 14 may be constructed integrally instead of with separate devices. In this case, each of the devices of the blood purification unit 14 are directly connected to the CPU bus of the control apparatus 50, sending and receiving information through the CPU bus to and from the first processing device 56 of the control apparatus 50.

The learning model may also learn by a method other than reinforcement learning. For example, the learning model may be trained in advance by supervised learning such as deep learning. In this case the teacher data used are multiple blood information items, and control parameters allowing stabilization of the state of blood that is represented by each blood information item. The learning model learns so that, when each blood information item has been input, it outputs control parameters that allow stabilization of the state of blood represented by each blood information item. The learning model may also learn by unsupervised learning, semi-supervised learning, transduction or multitask learning.

A blood purification device having apheresis function may be used as the blood purification device 1. Further, in the blood purification unit 14, a set of a blood purification device to separate blood plasma components and cell components from blood, and a device to separate factor components which are pathogenic factors from separated blood plasma components may be used. In the blood purification unit 14, a device to separate factor components which are pathogenic factors are provided on the upstream side or the downstream side of the blood purification device 1 (dialyzer). Thus, the blood purification system 40 may be used for apheresis. When the blood purification system 40 is used for apheresis, it can analyze and provide operation conditions that can reduce pathogenic substances.

REFERENCE SIGNS LIST

1 Blood purification device, 3 blood supply line, 4 blood return line, 5 blood pump, 6 resistance member, 7 replacement fluid container, 8 filtrate line, 9 filtration pump, 10 dialysate line, 11 dialysate pump, 12 fluid replacement line, 13 fluid replacement pump, 14, 14-2, 14-3, 14-4 blood purification unit, 21-23 first pressure gauge, 25 second pressure gauge, 26 third pressure gauge, 27 fourth pressure gauge, 28, 29 first flow meter, 30 second flow meter, 31 third flow meter, 32 fourth flow meter, 40 blood purification system, 50 control apparatus, 53 first communication device, 55 first storage device, 551 learning model, 561 first data acquisition module, 562 first generator, 563 first output control module, 564 parameter acquisition module, 565 control module, 80 server, 81 second communication device, 821 learning model, 831 second data acquisition module, 832 second generator, 833 second output control module.

**Claims**

1. A blood purification system comprising:

    a line through which a liquid containing blood or filtrate flows;
    a blood purification device to purify the blood flowing through the line;
    a supply device to supply dialysate or replacement fluid to the line;
    a detector to detect blood information relating to the blood flowing through the line;
    a liquid control mechanism to control flow of liquid in the line based on control parameters;

a parameter acquisition module to input the blood information detected by the detector into a learning model trained to output predetermined control parameters when predetermined blood information is input, and acquires control parameters outputted from the learning model; and

a control module to control the liquid control mechanism based on the control parameters acquired by the parameter acquisition module, wherein

the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

2. The blood purification system according to claim 1, wherein:

the learning model has an action value function using the blood information as a state, and control based on the control parameter as an action, and wherein

the action value function is updated based on a reward set so as to be greater as change in the blood information is smaller.

3. The blood purification system according to claim 1 or 2, wherein:

the liquid control mechanism includes a magnetic regulator to apply a magnetic field to blood in a predetermined direction, a pump to control the flow of liquid in the line, or a resistance member to apply resistance to the line, and wherein

the control parameters include at least one from among magnetic field strength applied by the magnetic regulator, drive amount of the pump and a value of resistance applied by the resistance member.

4. The blood purification system according to any one of claims 1 to 3, further comprising a storage to store the learning model in association with product data for the blood purification device, data relating to blood flowing through the line, clearance data by the blood purification system, causative substance removal, or data related to antithrombogenicity.

5. The blood purification system according to any one of claims 1 to 4, further comprising a communication device to receive the learning model from a learning apparatus.

6. The blood purification system according to any one of claims 1 to 4, further comprising a generation module to control the liquid control mechanism based on a specific control parameter, and generate the learning model based on the blood information detected by the detector before and after controlling the liquid control mechanism, and the specific control parameter.

7. The blood purification system according to any one of claims 1 to 6, wherein the blood purification system is an extracorporeal circulation blood purification system.

8. The blood purification system according to claim 7, wherein the blood purification system carries out continuous hemodialysis filtration, continuous hemofiltration, continuous hemodialysis or apheresis.

9. A control method of a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, comprising:

inputting the blood information detected by the detector into a learning model trained to output predetermined control parameters when predetermined blood information is input, and acquiring control parameters outputted from the learning model; and

controlling the liquid control mechanism based on the acquired control parameters, wherein

the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

10. A control program of a computer included in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, wherein the computer program causes the computer to execute a process, the process comprising:

inputting the blood information detected by the detector into a learning model trained to output predetermined control parameters when predetermined blood information is input, and acquiring control parameters outputted from the learning model; and
controlling the liquid control mechanism based on the acquired control parameters, wherein
the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

11. A learning apparatus comprising:

a data acquisition module to acquire, in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters;
a generation module to generate a learning model trained to output predetermined control parameters when predetermined blood information is input, using the sets acquired by the a data acquisition module; and
an output control module to output information relating to the learning model, wherein
the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

12. The learning apparatus according to claim 11,

further comprising a communication device to communicate with multiple blood purification systems,
wherein the data acquisition module acquires the sets by receiving it from the multiple blood purification systems via the communication device.

13. The learning apparatus according to claim 11, wherein the data acquisition module controls the liquid control mechanism based on specific control parameters, and acquires blood information detected by the detector before and after control of the liquid control mechanism, based on the specific control parameter, to acquire the sets.

14. A learning method comprising:

acquiring, by a computer, in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters;
generating, by the computer, a learning model trained to output predetermined control parameters when predetermined blood information is input, using the acquired sets; and
outputting, by the computer, information relating to the learning model, wherein
the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

15. A control program of a computer, wherein the computer program causes the computer to execute a process, the process comprising:

acquiring in a blood purification system including a line through which a liquid containing blood, filtrate flows, dialysate or replacement fluid, a blood purification device to purify the blood flowing through the line, a supply device to supply dialysate or replacement fluid to the line, a detector to detect blood information relating to the blood flowing through the line, a liquid control mechanism to control flow of liquid in the line based on control parameters, a plurality of sets of the blood information and the control parameters;

generating a learning model trained to output predetermined control parameters when predetermined blood information is input, using the acquired sets; and

outputting information relating to the learning model, wherein

the blood information includes at least one from among degree of blood flow turbulence, blood vorticity, degree of cardiac murmur, blood pressure, blood pressure loss, filtrate pressure, dialysate pressure, replacement fluid pressure, blood flow, filtrate flow, dialysate flow, replacement fluid flow, fouling, blood hematocrit value, degree of hemolysis and blood pressure of a patient connected to the line.

FIG. 1

# FIG. 2

Dialysate/replacement fluid
(with buffering action)

CHDF

Filtrate

Dialysate

Replacement fluid

FIG. 3

EP 4 129 354 A1

# FIG. 4

| Measurement ID | Learning model | Patient data | Product data | Blood data | Clearance data | Causative substance removal | Antithrombogenicity | ... |
|---|---|---|---|---|---|---|---|---|
| A00 | B001 | C001, C002,... | D001, D002,... | E001, E002,... | E001, E002,... | F001, F002,... | G001, G002,... | ... |
| A01 | B001 | C011, C012,... | D011, D012,... | E011, E012,... | E011, E012,... | F011, F012,... | G011, G012,... | ... |
| ... | ... | ... | ... | ... | ... | ... | ... | ... |

553

FIG. 5

EP 4 129 354 A1

# FIG. 6

```
        ( Learning processing )
                  │
    ┌─────────────▼─────────────┐
    │   Acquire blood information │───S101
    └──────────────┬────────────┘
                   ▼
    ┌───────────────────────────┐
    │   Acquire control parameter │───S102
    └──────────────┬────────────┘
                   ▼
    ┌───────────────────────────┐
    │         Line control       │───S103
    └──────────────┬────────────┘
                   ▼
    ┌───────────────────────────┐
    │   Acquire blood information │───S104
    └──────────────┬────────────┘
                   ▼
    ┌───────────────────────────┐
    │         Set reward         │───S105
    └──────────────┬────────────┘
                   ▼
    ┌───────────────────────────┐
    │  Update action value function │───S106
    └──────────────┬────────────┘
                   ▼
              ◇ Completion
       No    conditions satisfied ───S107
    ◄─────────      ?
                   │ Yes
                   ▼
    ┌───────────────────────────┐
    │      Generate and store    │───S108
    │        learning model      │
    └──────────────┬────────────┘
                   ▼
    ┌───────────────────────────┐
    │      Send learning model   │───S109
    └──────────────┬────────────┘
                   ▼
               ( END )
```

# FIG. 7

```
        ( Control processing )
                  │
    ┌────────────►│
    │             ▼
    │   ┌─────────────────────────┐
    │   │ Acquire blood information │──  S201
    │   └─────────────────────────┘
    │             ▼
    │   ┌─────────────────────────┐
    │   │ Acquire control parameter │──  S202
    │   └─────────────────────────┘
    │             ▼
    │   ┌─────────────────────────┐
    │   │      Control line        │──  S203
    │   └─────────────────────────┘
    │             ▼
    │   ┌─────────────────────────┐
    │   │ Acquire blood information │──  S204
    │   └─────────────────────────┘
    │             ▼
    │   ┌─────────────────────────┐
    │   │       Set reward         │──  S205
    │   └─────────────────────────┘
    │             ▼
    │   ┌─────────────────────────┐
    │   │ Update action value function │──  S206
    │   └─────────────────────────┘
    │             ▼
    │          ╱────────╲
    │    No   ╱ Completion ╲       S207
    └────────◄ indication received ►
             ╲      ?      ╱
              ╲──────────╱
                  │ Yes
                  ▼
        ┌─────────────────────┐
        │ Acquire and store data │── S208
        └─────────────────────┘
                  ▼
        ┌─────────────────────┐
        │   Display results    │── S209
        └─────────────────────┘
                  ▼
             (   END   )
```

27

# FIG. 8

Learning processing

Acquire set of
blood information and
control parameter — S301

Update action value function — S302

Set reward — S303

Generate and store
learning model — S304

Send learning model — S305

END

# FIG. 9

# FIG. 10

Dialysate/replacement fluid
(with buffering action)

CHDF

14-3

7

24

28

5

21 Filtrate

8

1c

17

16

1

1e

1b

1a

3

25

30

Dialysate

10

11

23

26

31

1d

24'

22

13

18

Replacement fluid

12

29

27

32

4

2

6

Blood
(with buffering action)

(with resistance)

# FIG. 11

Dialysate/replacement fluid
(with buffering action)

CHD
CHF

14-4

28

24

21

5

1b Filtrate

1c

17

16

1 1e

3

23

2

1d

24'

22

4

29

18

Blood
(with buffering action)

6
(with resistance)

7

8

25 30

9

11

10

1a Dialysate

26 31

**INTERNATIONAL SEARCH REPORT**

| International application No. |
| --- |
| PCT/JP2021/014033 |

**A. CLASSIFICATION OF SUBJECT MATTER**
Int. Cl. A61M1/02(2006.01)i, A61M1/16(2006.01)i
FI: A61M1/16 110, A61M1/16 111, A61M1/16 115, A61M1/16 117, A61M1/02 100

According to International Patent Classification (IPC) or to both national classification and IPC

**B. FIELDS SEARCHED**

Minimum documentation searched (classification system followed by classification symbols)
Int. Cl. A61M1/02, A61M1/16

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2014-518692 A (MEDTRONIC INC.) 07 August 2014, | 1, 3-15 |
| Y | paragraphs [0024]-[0027], [0034]-[0044], [0055]-[0069], [0079]-[0094], fig. 1-7, 10-15 | 2 |
| Y | WO 2020/027174 A1 (DAINIPPON PRINTING CO., LTD.) 06 February 2020, paragraph [0046] | 2 |
| A | US 2018/0361051 A1 (FRESENIUS MEDICAL CARE DEUTSCHLAND GMBH) 20 December 2018, entire text, all drawings | 1-15 |
| A | US 2017/0216513 A1 (FRESENIUS MEDICAL CARE DEUTSCHLAND GMBH) 03 August 2017, entire text, all drawings | 1-15 |

☐ Further documents are listed in the continuation of Box C.   ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 13.05.2021 | 25.05.2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/014033

| Patent Documents referred to in the Report | Publication Date | Patent Family | Publication Date |
|---|---|---|---|
| JP 2014-518692 A | 07.08.2014 | WO 2012/148790 A1 paragraphs [0027]-[0032], [0039]-[0049], [0060]-[0074], [0084]-[0099], fig. 1-7, 10-15<br>US 2012/0277722 A1<br>CN 103747819 A | |
| WO 2020/027174 A1 | 06.02.2020 | (Family: none) | |
| US 2018/0361051 A1 | 20.12.2018 | WO 2017/102553 A1 entire text, all drawings<br>DE 102015016271 A1<br>CN 108391448 A | |
| US 2017/0216513 A1 | 03.08.2017 | WO 2016/012093 A1 entire text, all drawings<br>DE 102014010907 A1<br>CN 106659832 A | |

Form PCT/ISA/210 (patent family annex) (January 2015)

**REFERENCES CITED IN THE DESCRIPTION**

**Patent documents cited in the description**

- JP 2012249748 A